# EUROPÄISCHE PATENTANMELDUNG

(11) **EP 1 629 862 A1**
(43) Veröffentlichungstag der Anmeldung: **01.03.2006**
(21) Anmeldenummer: 05018902.6
(22) Anmeldetag: 31.08.2005
(51) Int. Cl.: A61M 39/02

(54) **Subcutan implantierbarer Infusionsport**

(30) Priorität: 31.08.2004 DE 102004042076
(71) Anmelder: Steco-System-Technik GmbH & Co. KG, 22529 Hamburg (DE)
(72) Erfinder: Krug, Dr. Florian, 22526 Hamburg (DE); Münder, Dipl.-Ing. Ulrich, 23556 Lübeck (DE); Kuhlemann, Jörg, 32694 Dörentrup (DE); Schön, Manfred, 32694 Dörentrup (DE)
(74) Vertreter: Klingseisen, Franz

(57) **Zusammenfassung**

Es wird ein subcutan implantierbarer Infusionsport vorgeschlagen, umfassend ein Portgehäuse (2) und eine durch Kanülen perforierbare, elastische Portmembran (10), die in einen konischen Innenraum des Portgehäuses (2) eingesetzt ist, wobei die Portmembran (10) auf der proximalen Seite den größten Durchmesser aufweist und an einem Absatz (7) auf dem Innenumfang des Portgehäuses (2) anliegt, wobei das Portgehäuse (2) einen rotationssymmetrischen Membransitz (8) aufweist, und die Portmembran (10) ebenfalls rotationssymmetrisch ausgebildet ist, wobei sie im Durchmesser durchgehend größer ausgelegt ist als der Durchmesser des Membransitzes (8), so dass sich eine radiale Verpressung der Portmembran (10) nach dem Einsetzen in den Membransitz (8) ergibt, und die Portmembran (10) auf der distalen Seite eine konkave Stirnfläche (11) aufweist.

## Beschreibung

Die Erfindung betrifft einen subcutan implantierbaren Infusionsport nach dem Oberbegriff des Anspruchs 1. Ein derartiger Infusionsport wird für die kontinuierliche oder intermittierende Zufuhr von Medikamenten, für Blutentnahme bzw. Blutzufuhr oder zum Auffüllen von Gewebeexpandern bzw. für andere hydraulische Implantatsysteme verwendet.

Ein Infusionsport dieser Art ist aus DE 44 23 706 C1 bekannt, der ein kegelstumpfförmiges, auf einer Längsseite abgeflachtes Portgehäuse aufweist, wobei die Portmembran im eingesetzten Zustand im wesentlichen parallele Stirnflächen aufweist. Die Portmembran hat einen elliptischen Querschnitt entsprechend dem elliptischen Membransitz des elliptischen, kegelstumpfförmigen Residualraums im Portgehäuse. Dadurch, dass die distale Stirnseite der Portmembran im wesentlichen eine ebene Fläche etwa senkrecht zu den angrenzenden Wänden des Residualraums bildet, ergeben sich im Umfangsbereich strömungstechnische Toträume, wenn durch eine durch die Portmembran eingeführte Kanüle ein Fluid eingeführt wird.

Weiterhin ist ein derartiger Infusionsport aus DE 196 24 320 bekannt, wobei die Stirnseite der Portmembran auf der distalen Seite konkav und auf der proximalen Seite plan ausgebildet ist. Auf der distalen Seite geht die Konkavität der Portmembran mit einem spitz zulaufenden Umfangsrand in den konischen Residualraum über und schließt sich ohne Absatz an den konischen Residualraum des Portgehäuses an.

Der Erfindung liegt die Aufgabe zugrunde, einen Infusionsport der eingangs angegebenen Art so auszubilden, dass er für die klinische Anwendung optimiert ist.

Dies wird durch die Merkmale im kennzeichnenden Teil des Anspruchs 1 ereicht. Dadurch, dass eine rotationssymmetrische Portmembran unter radialer Verpressung in einen entsprechenden rotationssymmetrischen Membransitz eingesetzt ist, ergibt sich auf der proximalen Stirnseite der Portmembran eine gewölbte Stirnfläche, die mit der konkaven Stirnfläche auf der distalen Seite eine über den Querschnitt im wesentlichen gleichmäßige Durchstechtiefe für eine Kanüle bildet, während sich zugleich ein zuverlässiger und dauerhafter Dichtsitz der Portmembran im Portgehäuse ergibt und strömungstechnische Toträume auf der distalen Seite vermieden werden.

Eine beispielsweise Ausführungsform der Erfindung wird nachfolgend anhand der Zeichnung näher erläutert. Es zeigen
- Fig. 1: eine Draufsicht auf einen Infusionsport
- Fig. 2: einen teilweisen Längsschnitt des Portgehäuses und der Portmembran in nicht eingesetztem Zustand,
- Fig. 3: in der Darstellung in Fig. 2 die Portmembran im eingesetzten Zustand im Portgehäuse,
- Fig. 4: den subcutan implantierten Infusionsport,
- Fig. 5: eine abgewandelte Ausführungsform der Portmembran in einer Seitenansicht,
- Fig. 6: in einem Längsschnitt den Katheteranschluss, und
- Fig. 7: einen Längsschnitt durch das Portgehäuse.
- Fig. 8: einen Längsschnitt durch eine andere Formgebung des Portgehäuses längs der Linie A-A in Fig. 10,
- Fig. 9: eine Seitenansicht des Portgehäuses nach Figur 8,
- Fig. 10: eine Draufsicht auf das Portgehäuse nach Fig. 9, und
- Fig. 11: eine Stimansicht des Portgehäuses nach Fig. 9 und 10

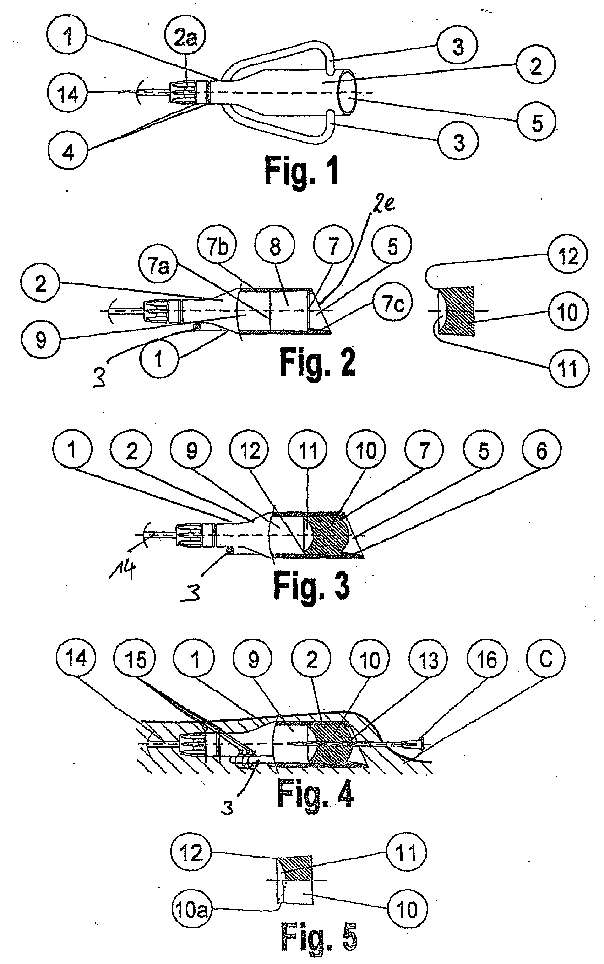

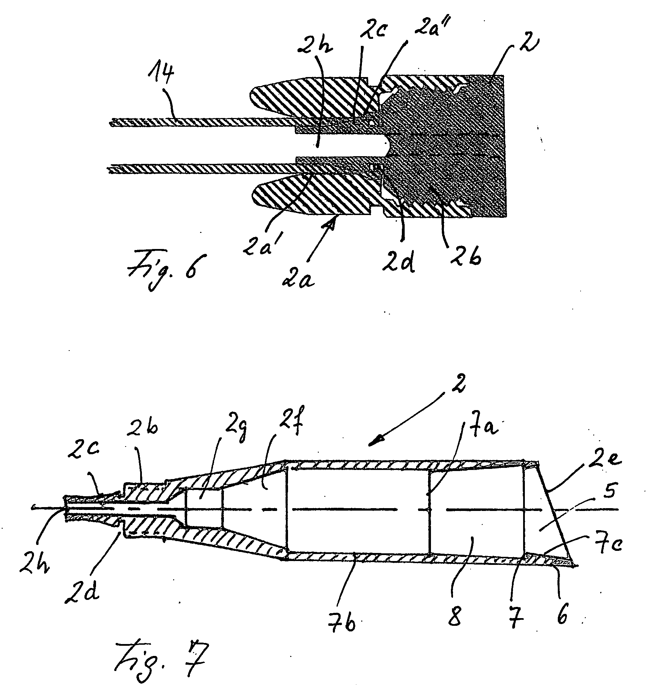

Der in Fig. 1 wiedergegebene Infusionsport 1 weist ein im wesentlichen rohrförmiges, rotationssymmetrisches Portgehäuse 2 auf, an dessen beiden Seiten Stabilisationsbügel 3 angebracht sind, die auf der proximalen Seite etwas oberhalb der Längsachse des Portgehäuses angesetzt sind und schräg zur Längsachse zur distalen Seite verlaufen, wobei sie, wie Fig. 2 zeigt, unter dem sich verjüngenden Abschnitt des Portgehäuses 2 miteinander verbunden sind. An dem im Durchmesser verjüngten distalen Abschnitt des Portgehäuses 2 sind in Fig. 1 Umfangsnuten 4 ausgebildete, die dazu dienen, bei der Implantation ein Nahtmaterial aufzunehmen, das um das Portgehäuse geschlungen wird. Diese Nahtnuten 4 können alternativ oder ergänzend zur Nahtfixation 15 (Fig. 4) am Stabilisationsbügel 3 vorgesehen sein. Mit 14 ist in Fig. 1 ein Katheter bezeichnet, der mit einem in dem Portgehäuse 2 ausgebildeten Residualraum 9 in Verbindung steht. An dem im Durchmesser verjüngten distalen Ende des Portgehäuses 2 ist eine Kathetersicherungshülse 2a aufgeschraubt (Fig. 6). Das Portgehäuse 2 besteht aus Metall wie Titan oder auch aus einem entsprechenden festen Kunststoff.

Wie Fig. 2 zeigt, ist die proximale Stirnseite 2e des Portgehäuses 2 relativ zur Längsachse abgeschrägt. Senkrecht zur Längsachse verläuft nahe der Stirnseite ein Absatz 7 auf dem Innenumfang des rohrförmigen Portgehäuses. Von diesem Absatz 7 aus erstreckt sich ein rotationssymmetrischer, kegelstumpfförmiger Membransitz 8 in einem Winkel von unter 10° relativ zur Längsachse. Der maximale Durchmesser des Membransitzes 8 liegt auf der proximalen Seite angrenzend an den Absatz 7. An dem mit 7a bezeichneten Ende des konischen Membransitzes 8 geht dieser ohne Absatz in einem im wesentlichen zylindrischen Abschnitt 7b über, worauf sich das Portgehäuse, wie dargestellt, verjüngt und in den Katheteransatz übergeht. Auf der proximalen Seite hat die Innenumfangsfläche des Portgehäuses 2 im Eingangsbereich 5 zwischen Absatz 7 und proximaler Stirnseite 2e vorzugsweise ebenfalls einen Neigungswinkel relativ zur Längsachse etwa in der Größenordnung von etwa 10° derart, dass die Innenumfangsfläche sich zur proximalen Seite hin erweitert.

Wie Fig. 2 und 3 zeigen, ergibt sich auf der dem Muskelgewebe zugewandten unteren Seite des Portgehäuses 2 durch die schräge Stirnfläche relativ zu dem Absatz 7 eine in proximaler Richtung verlängerte untere Gehäuselippe 6, durch die das Gewebe unter dem proximalen Ende vor einem zu steilen Einstich einer Kanüle geschützt wird, die in den Residualraum 9 eingeführt werden soll.

Ein in Fig. 2 in unverpresstem Zustand wiedergegebene Portmembran 10 weist eine rotationssymmetrische konische Umfangsfläche mit einem Neigungswinkel unter 10° entsprechend dem Membransitz 8 im Portgehäuse 2 auf, wobei die proximale Stirnseite im wesentlichen senkrecht zur Längsachse verläuft und die distale Stirnseite bei 11 konkav ausgebildet ist, derart, dass sich zwischen distalem Außenrand der Portmembran 10 und Innenrand der Konkavität 11 eine ringförmige Stirnfläche 12 ergibt, deren Ebene etwa senkrecht zur Längsachse liegt.

Die Portmembran 10 besteht vorzugsweise aus Silikon und sie hat eine Shore-Härte A von 30 bis 80, vorzugsweise 50. Ihr Durchmesser ist größer ausgelegt als der des Membransitzes 8, vorzugsweise in der Größenordnung von 2 bis 10%, wobei bei einer Shore-Härte von A 30 das Übermaß der Portmembran etwa 10% und bei einer Shore-Härte von A 70 das Übermaß etwa 2% beträgt.

Der vorzugsweise konstante Winkel der Koniziät am Membransitz 8 und am Außenumfang der Portmembran 10 ist bevorzugt so ausgelegt, dass er selbstsichernd ist, d.h. dass die Portmembran durch die Reibungskraft zwischen Membranoberfläche und Oberfläche des Membransitzes sicher gehalten wird, wenn von der proximalen Seite aus mittels einer Kanüle ein Druck auf die Portmembran 10 ausgeübt wird.

Der Winkel des Membransitzes 8 und der Konizität der Portmembran 10 ist vorzugsweise 5 bis 8° und insbesondere 7°.

Es ist auch möglich, die Fläche des Membransitzes 8 bspw. durch wenigstens abschnittsweises Aufrauhen so zu gestalten, dass die Reibung der Portmembran erhöht wird.

Fig. 3 zeigt die in das Portgehäuse 2 eingesetzte Portmembran 10. Aufgrund des radialen Übermaßes der Portmembran 10 von 2 bis 10 % gegenüber dem Durchmesser des Membransitzes 8 und der elastischen Eigenschaften des Silikonmaterials der Portmembran 10 ergibt sich auf der proximalen Stirnseite eine konvex geformte Fläche durch die radiale Verpressung, während auf der distalen Seite ein Absatz auf dem Innenumfang des Portgehäuses zur Halterung der Portmembran nicht erforderlich ist. Die Konkavität 11 auf der distalen Stirnseite der Portmembran ist so ausgelegt, dass sich bei der radialen Verpressung und der dadurch hervorgerufenen konvexen Stirnseite auf der proximalen Seite eine im wesentlichen gleiche Durchstechtiefe in Achsrichtung ergibt, so dass beim Einstechen einer Kanüle 16 (Fig. 4), die bei üblicher Einstechrichtung etwa parallel zur Achse des Portgehäuses bzw. parallel zur Hautoberfläche geführt wird, im wesentlichen immer der gleiche Widerstand auftritt, unabhängig von der Einstichstelle auf dem Querschnitt der Portmembran. Dadurch, dass auf der distalen Seite kein Absatz zur Halterung der Portmembran im Portgehäuse vorhanden ist, besteht auch keine Gefahr, dass beim Einstechen einer Kanüle unter einem ungünstigen Winkel diese gegen einen solchen Absatz stößt und eine Infusion behindert wird.

Der Übergang von der Konkavität 11 auf der distalen Stirnseite der Portmembran 10 auf den im wesentlichen zylindrischen Innenumfang des Residualraumes 9 verhindert das Entstehen von strömungstechnischen Toträumen.

Die ringförmige Stirnfläche 12 zwischen Außenumfang der Portmembran 10 und Innenumfang der Konkavität 11 gewährleistet einen ausreichenden Anpressdruck des Silikonmaterials auf dem distalen Bereich des Membransitzes 8, um die Dichtigkeit zu gewährleisten. Bei einem spitz zulaufenden Umfangsrand auf der distalen Seite der Portmembran 10 würde sich kein ausreichender Anlagedruck radial nach außen an der Portmembran 10 ergeben. Dadurch, dass der Randbereich der Portmembran angrenzend an die ringförmige Stirnfläche 12 eine größere Materialstärke aufweist, ergibt sich ein ausreichender Anpressdruck an dem Membransitz 8 in diesem Bereich zur Gewährleisung eines dichten Sitzes.

Durch die radiale Verpressung über die Länge der Portmembran ergibt sich eine gleichbleibende Dauerdichtigkeit über die ganze Länge der Portmembran, die auch durch mehrfache Punktion nicht beeinträchtigt wird. Des weiteren werden durch die auf gesamter Länge dauerhaft und radial auf die Portmembran 10 wirkenden Kräfte die Punktionskanülen gegen ein unbeabsichtigtes Herausziehen gesichert.

Bei der Montage der Portmembran 10 wird dies über den ringförmigen Absatz 7 in das Portgehäuse 2 bzw. in den konischen Membransitz 8 eingepresst. Vorzugsweise wird anschließend der Residualraum 9 vom katheterseitigen Ende mit einem Überdruck beaufschlagt, um die Portmembran gegen den Absatz 7 zu pressen, wodurch eine zusätzliche axiale Verpressung hervorgerufen wird und die Dichtigkeit des Residualraumes 9 bei stark erhöhtem Innendruck gewährleistet wird. Auf diese Weise wird die Portmembran 10 dauerhaft radial und axial verpresst, wobei die axiale Verpressung geringer ist als die radiale Verpressung.

Die Portmembran 10 hat ein Durchmesser-Längen-Verhältnis von 1,0 bis 2,0 und vorzugsweise von 1,5, um die hauptsächlich radiale Verpressung auf ganzer Membranlänge gleichmäßig zu gewährleisten.

Fig. 4 zeigt den subcutan implantierten Infusionsport mit etwa parallel zur Hautoberfläche eingestochener Kanüle 16, wobei bei C das Gewebe schematisch wiedergegeben ist. Der Infusionsport 1 wird derart subcutan implantiert, dass eine Kanüle 16 im wesentlichen parallel zur Hautoberfläche eingeführt werden kann, wie dies Fig. 4 zeigt. Der Infusionsport wird durch Nahtmaterial 15, das aus resorbierbarem Material bestehen kann, im Gewebe fixiert, wobei in Fig. 4 die Fäden um den Bügel 3 und um den im Durchmesser verjüngten distalen Abschnitt des Portgehäuses 2 sowie um die Kathetersicherungshülse 2a in den Nuten 4 geführt sind, wie dies Fig. 4 zeigt. Dadurch, dass der Infusionsport auf der distalen Seite durch Nahtmaterial fixiert ist, ergibt sich eine gewisse Schwenkbarkeit des Infusionsports 1 relativ zur Hautoberfläche. Hierdurch ergibt sich ein außergewöhnlich großes, kreisausschnittförmiges Punktionsfeld für das Ansetzen einer Kanüle 16, so dass das Hautgewebe hinsichtlich der Punktionsbeeinträchtigung stark entlastet wird. Ferner ist der lange, subcutane Punktionskanal zwischen proximaler Stirnseite des Infusionsports und Hautoberfläche geeignet, Infektionen vorzubeugen, da über die Kanüle eindringende Keime eine sehr lange Distanz überwinden müssen, die eine verbesserte Infektionssperre darstellt.

Mit 5 ist der in der Querschnittsansicht in den Fig. 1 bis 3 sich nach unten verbreiternde Eingangsbereich des Portgehäuses 2 vor der proximalen Stirnseite der Portmembran 10 bezeichnet. Die an der Unterseite ausgebildete Lippe 6 dient dazu, die Muskelfacies vor einer unbeabsichtigten Punktion mit einer Kanüle 16 zu schützen, die bei einer Fehlstellung der Kanüle schräg von oben eingestochen werden könnte. Die Neigung der Innenumfangsfläche 7c der Lippe 6 von der Stirnseite 2e aus radial nach innen unterstützt dabei die Führung der Kanüle in Richtung auf die Portmembran 10, die, wie Fig. 4 zeigt, etwa parallel zur Hautoberfläche eingestochen werden sollte.

Bei der Implantation des Infusionsports 1 wird dieser mit der Portmembran 10 voran in eine Gewebetasche geschoben, bis nur noch das hintere Drittel mit dem Katheteranschluss sichtbar ist und somit auch nur der hintere, katheterseitige Bereich für manuelle Arbeiten der Fixierung zugänglich ist.

Fig. 6 zeigt in einem Längsschnitt im Einzelnen die Verbindung des Katheters 14 mit dem Portgehäuse 2. Die Kathetersicherungshülse 2a ist auf einen Gewindeabschnitt 2b des Portgehäuses aufgeschraubt, an den in distaler Richtung ein hülsenförmiger Ansatz 2c angeformt ist, der auf dem Außenumfang einen in proximaler Richtung sich vergrößernden Durchmesser aufweist. Der Katheter 14 wird über diesen Ansatz 2c geschoben, bis der Rand des Katheters 14 in eine Umfangsnut 2d zwischen Gewindeabschnitt 2b und dem konischen Abschnitt des Ansatzes 2c zu liegen kommt. Hierauf wird die Kathetersicherungshülse 2a aufgeschraubt, die auf dem Innenumfang einen zylindrischen Abschnitt 2a' aufweist, mittels dem das Material des Katheters 14 an dem konischen Abschnitt des Ansatzes 2c festgeklemmt wird. In proximaler Richtung schließt sich an den zylindrischen Abschnitt 2a' eine konische Erweiterung 2a" auf dem Innenumfang der Sicherungshülse 2a an, so dass sich das Material des Katheters 14 im Bereich der Umfangsnut 2d wieder entspannen kann. Das distale Ende der Sicherungshülse 2a ist zur Erleichterung des Einführens des Katheters 14 nach außen erweitert bzw. konisch gestaltet und mit einer abgerundeten distalen Stirnseite versehen, wie dies Fig. 6 zeigt.

Fig. 7 zeigt in einem schematischen Längsschnitt eine praktische Ausgestaltung eines Portgehäuses 2. An den konischen Membransitz 8 schließ sich der zylindrische Abschnitt 7b an, der den Residualraum 9 begrenzt. In distaler Richtung anschließend an den zylindrischen Abschnitt 7b ist ein verstärkt konischer Abschnitt 2f vorgesehen, der in einem zylindrischen Abschnitt 2g kleineren Durchmessers übergeht, der wiederum über einen konischen Abschnitt in die Bohrung 2h am distalen Ende des Portgehäuses 2 übergeht. Die rotationssymmetrische Ausgestaltung des gesamten Portgehäuses 2, bei der die einzelnen Hohlraumabschnitte 5, 8, 7b, 2f, 2g und 2h koaxial zueinander ausgebildet sind und sich ein in Richtung auf die distale Bohrung 2h hin verjüngender Aufbau ergibt, begünstigt die Einführung von Instrumenten durch die Portmembran 10 in den Katheter 14, wie z.B. von mikrochirurgischen Instrumenten, Sonden oder Bürsten zur Reinigung des Katheters. Durch die röhrenförmige Ausgestaltung des Innenraums mit sich zur Bohrung 2h hin verjüngenden Abschnitten ergibt sich für ein einzuführendes Instrument kein Hindernis, wobei die konischen Abschnitte ähnlich einem Trichter zur Führung in Richtung Katheteröffnung dienen. Die Übergänge zwischen den einzelnen Abschnitten 7b, 2f, 2g sind gerundet ausgebildet, so dass sich keine Ecken ergeben.

Durch die sich nach außen in proximaler Richtung erweiternde Innenumfangsfläche 7c und die sich ergebenden dünnen Wandbereiche des Portgehäuses 2 an der proximalen Stirnseite 2e ergibt sich eine optimale Exposition der Portmembran 10 für einen leichten Zugang einer Injektionsnadel, wobei die im unteren Bereich weiter in proximale Richtung vorstehende Lippe 6 einen Schutz des darunter liegenden Gewebes bildet.

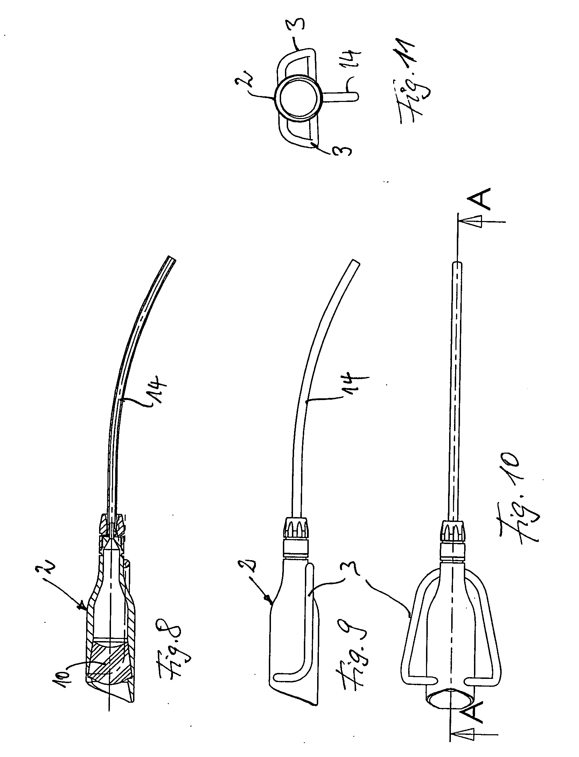

Fig. 8 bis 11 zeigen Ansichten des Portgehäuses 2 mit Katheter 14, wobei die abgewinkelte Form des Stabilisationsbügels 3 deutlich erkennbar ist, der in der Stirnansicht nach Fig. 11 eine das Portgehäuse abstützende Funktion hat.

Es sind verschiedene Abwandlungen der beschriebenen Ausführungsform möglich. So kann sich am distalen Randbereich der Portmembran 10 an den konischen Außenumfang ein im wesentlichen zylindrischer Abschnitt 10a anschließen, wie dies Fig. 5 zeigt, der durch die ringförmige Stirnfläche 12 begrenzt wird. Da der Membransitz 8 durchgehend konisch ausgebildet ist, wird durch den zylindrischen Abschnitt ein zusätzlicher Anpressdruck im distalen Endbereich erreicht.

Weiterhin kann der Residualraum 9 angrenzend an das distale Ende des Membransitzes 8 auch konisch in Richtung auf den Katheteransatz ausgebildet sein. Eine etwa zylindrische Ausgestaltung, wie bei 7b in Fig. 2 und Fig. 7 dargestellt, beugt der Gefahr eines Kontaktes und einer Beschädigung der Kanülenspitze an der Portinnenwand vor und begünstigt eine verbesserte Spülbarkeit des Residualraumes nach Beendigung einer Infusion und verhindert mit der konkaven Rückseite 11 der Portmembran 10 und der Ringfläche 12 Ablagerungen von Medikamentenausfällungen.

Die Stabilisationsbügel 3 haben vorzugsweise einen kreisrunden Querschnitt, und es können anstelle der Stabilisationsbügel 3 auch kompakte, in sich geschlossene Stabilisationsbleche vorgesehen werden, die dem im wesentlichen rotationssymmetrischen Portgehäuse 2 in der Draufsicht und in der Seitenansicht eine trapezförmige bzw. dreieckige Kontur verleihen, die die Gewebespannung der darüber liegenden Haut entlastet. Die offenzellige Struktur der Stabilisationsbügel 3 begünstigt eine bindegewebige Einscheidung zur postoperativen Sekundärfixation des Infusionsportes 1.

Weiterhin kann ein Doppelinfusionsport in der Weise ausgeführt werden, dass zwei nebeneinander liegende Infusionsgehäuse 2 miteinander verbunden werden und ein Fixationsbügel 3 nur auf den Außenseiten der beiden Portgehäuse 2 vorgesehen wird. Ein derartiger Doppelinfusionsport ist bei einigen medizinischen Anwendungen erforderlich, wobei beide Infustionsporte in der beschriebenen Weise ausgestaltet sind.

## Patentansprüche

1. Subcutan implantierbarer Infusionsport, umfassend
ein Portgehäuse (2) und eine durch Kanülen perforierbare, elastische Portmembran (10), die in einen konischen Innenraum des Portgehäuses eingesetzt ist, wobei die Portmembran auf der proximalen Seite den größten Durchmesser aufweist und an einem Absatz (7) auf dem Innenumfang des Portgehäuses (2) anliegt,
**dadurch gekennzeichnet,**
**dass** das Portgehäuse (2) einen rotationssymmetrischen Membransitz (8) aufweist, und die Portmembran (10) ebenfalls rotationssymmetrisch ausgebildet ist, wobei sie im Durchmesser durchgehend größer ausgelegt ist als der Durchmesser des Membransitzes (8), so dass sich eine radiale Verpressung der Portmembran nach dem Einsetzen in den Membransitz (8) ergibt, und dass die Portmembran (10) auf der distalen Seite eine konkave Stirnfläche (11) aufweist.

2. Infusionsport nach Anspruch 1,
wobei der Winkel des Membransitzes (8) sowie der Winkel der Portmembran (10) relativ zur Längsachse unter 10° liegt und vorzugsweise 5 bis 8 °, insbesondere 7° beträgt.

3. Infusionsport nach Anspruch 1,
wobei der Durchmesser der Portmembran (10) im unverpressten Zustand 2 bis 10% größer ist als der Durchmesser des Membransitzes (8).

4. Infusionsport nach einem der vorhergehenden Ansprüche,
wobei die Portmembran (10) im eingesetzten Zustand eine konvexe Stirnfläche im Eingangsbereich des Portgehäuses (2) aufweist, die mit der Konkavität (11) auf der distalen Stirnfläche in Achsrichtung eine im wesentlichen gleiche Durchstechtiefe über den Querschnitt der Portmembran ergibt.

5. Infusionsport nach einem der vorhergehenden Ansprüche,
wobei die Portmembran (10) eine Shore-Härte A von 30 bis 80, insbesondere 50 hat.

6. Portmembran nach Anspruch 1,
wobei die Portmembran (10) auf der distalen Seite zwischen Ende der konkaven Stirnfläche (11) und Außenumfang eine ringförmige Stirnfläche (12) aufweist.

7. Infusionsport nach Anspruch 6,
wobei die Portmembran (10) im distalen Endbereich auf dem Außenumfang einen zylindrischen Abschnitt (10a) aufweist, der sich an die konische Außenumfangsfläche anschließt.

8. Infusionsport nach einem der vorhergehenden Ansprüche,
wobei der konische Membransitz (8) auf der distalen Seite ohne Absatz in einen im wesentlichen zylindrischen Abschnitt (7b) übergeht.

9. Infusionsport nach einem der vorhergehenden Ansprüche,
wobei der Absatz (7) auf der proximalen Seite des Membransitzes (8) in einer Ebene etwa senkrecht zur Achse des Portgehäuses (2) liegt und das Portgehäuse auf der proximalen Seite eine schräg zur Längsachse liegende Stirnseite (2e) derart aufweist, dass sich auf der der Muskelfacies zugewandten Seite eine proximal vorstehende Portgehäuselippe (6) ergibt.

10. Infusionsport nach Anspruch 9,
wobei der Eingangsbereich (5) des Portgehäuses (2) vor der Portmembran (10) eine schräg zur Längsachse verlaufende, nach außen erweiterte Innenumfangsfläche (7c) aufweist.

11. Infusionsport nach einem der vorhergehenden Ansprüche,
wobei am katheterseitigen bzw. distalen Ende des Portgehäuses (2) umlaufende Nahtnuten (4) für eine Nahtfixation ausgebildet sind.

12. Infusionsport nach einem der vorhergehenden Ansprüche,
wobei auf dem Außenumfang des rohrförmigen Portgehäuses (2) im proximalen Bereich über der Längsachse auf gegenüberliegenden Seiten angesetzte Bügel (3) vorgesehen sind, die unter dem im Durchmesser verjüngten distalen Bereich des Portgehäuses miteinander verbunden sind .

13. Infusionsport nach einem der vorhergehenden Ansprüche,
wobei zwei nebeneinanderliegende Portgehäuse (2) miteinander verbunden sind und an den gegenüberliegenden Außenseiten der beiden Portgehäuse Stabilisationsbügel (3) ausgebildet sind.
